# EUROPEAN PATENT APPLICATION

(11) **EP 3 108 816 A2**
(43) Date of publication of application: **28.12.2016**
(21) Application number: 16172850.6
(22) Date of filing: 03.06.2016
(51) Int. Cl.: A61B 7/04, A61B 5/06, G06F 19/00

(54) **ELECTRONIC AUSCULTATION SYSTEM**

(30) Priority: 04.06.2015 JP 2015114233
(71) Applicant: Nihon Kohden Corporation, Shinjuku-ku Tokyo 161-8560 (JP)
(72) Inventor: Minegishi, Yuka, Tokyo, 161-8560 (JP); Ono, Yoshinobu, Tokyo, 161-8560 (JP)
(74) Representative: Hoffmann Eitle

(57) **Abstract**

An electronic auscultation system may include: an electronic stethoscope including a chest piece which is adapted to be in contact with a living body surface; a position acquiring section which is configured to acquire position information indicating a position of the chest piece with respect to the living body surface; a contact state acquiring section which is configured to acquire contact state information indicating a contact state of the chest piece with respect to the living body surface; and a recording section which is configured to record the position information and the contact state information while associating the position information with the contact state information.

## Description

### BACKGROUND

The presently disclosed subject matter relates to an electronic auscultation system in which an electronic stethoscope is used.

For example, JP-A-2007-135611 and JP-A-2013-123494 disclose systems in which biological information obtained from an electronic stethoscope is visualized to be used in diagnosis.

Auscultation is a skill which requires substantial clinical experience. In order to obtain correct biological information, predetermined auscultation conditions must be correctly reproduced. Auscultation conditions may be hardly reproduced not only in the case where the attending doctor is changed to another doctor, but also in the case where a person (e.g., a family member) other than the attending doctor handles a stethoscope in a situation such as remote medicine or home medical care.

### SUMMARY

The presently disclosed subject matter may enable auscultation conditions to be correctly reproduced regardless of a person who handles a stethoscope, and support diagnosis more correctly and multidirectionally by means of electronic auscultation.

There may be provided an electronic auscultation system comprising: an electronic stethoscope including a chest piece which is adapted to be in contact with a living body surface; a position acquiring section which is configured to acquire position information indicating a position of the chest piece with respect to the living body surface; a contact state acquiring section which is configured to acquire contact state information indicating a contact state of the chest piece with respect to the living body surface; and a recording section which is configured to record the position information and the contact state information while associating the position information with the contact state information.

### BRIEF DESCRIPTION OF THE DRAWINGS

Figs. 1 (A) and 1 (B) are views illustrating an electronic auscultation system of a first embodiment.
Fig. 2 is a view illustrating an example of a position acquiring section in the electronic auscultation system of Fig. 1.
Figs. 3 (A) and 3 (B) are views illustrating other examples of the position acquiring section in the electronic auscultation system of Fig. 1.
Figs. 4(A) and 4(B) are views illustrating examples of a guidance information providing section in the electronic auscultation system of Fig. 1.
Figs. 5 (A) and 5 (B) are views illustrating other examples of the guidance information providing section in the electronic auscultation system of Fig. 1.
Figs. 6(A) to 6(C) are views illustrating further examples of the guidance information providing section in the electronic auscultation system of Fig. 1.
Figs. 7(A) and 7(B) are views illustrating examples of a reproduction information providing section in the electronic auscultation system of Fig. 1.
Fig. 8 is a view illustrating an electronic auscultation system of a second embodiment.
Fig. 9 is a view illustrating an electronic auscultation system of a third embodiment.
Figs. 10(A) and 10(B) are views illustrating the operation of the electronic auscultation system of Fig. 9.
Figs. 11(A) and 11(B) are views illustrating the operation of the electronic auscultation system of Fig. 9.
Figs. 12(A) and 12(B) are views illustrating the operation of the electronic auscultation system of Fig. 9.

### DETAILED DESCRIPTION OF EXEMPLARY EMBODIMENTS

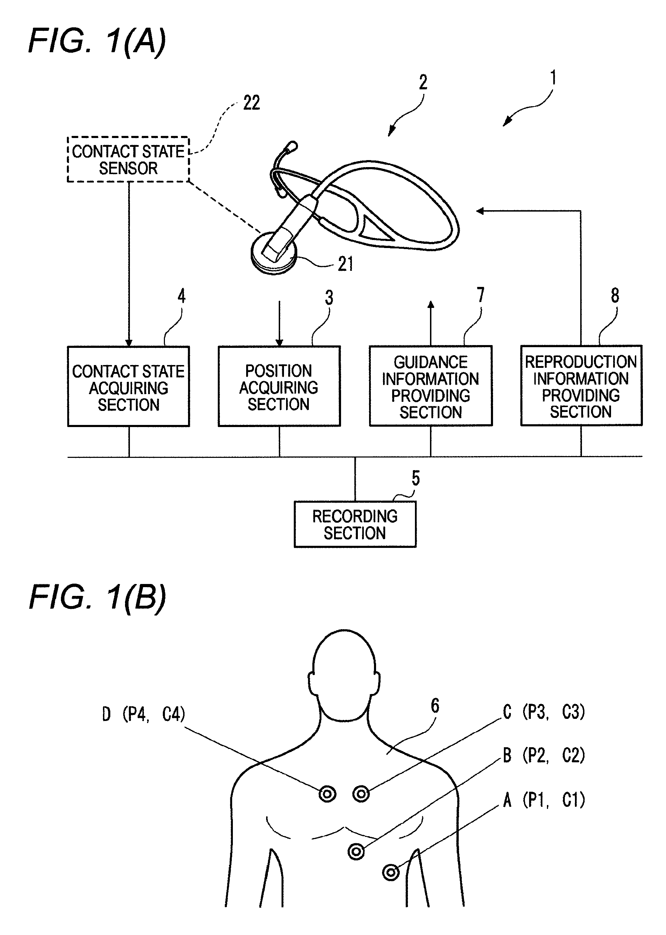

Hereinafter, embodiments will be described in detail with reference to the accompanying drawings. Fig. 1(A) diagrammatically illustrates the functional configuration of an electronic auscultation system 1 of a first embodiment.

The electronic auscultation system 1 may include an electronic stethoscope 2. The electronic stethoscope 2 may include a chest piece 21 which is caused to be in contact with the surface of the living body (hereinafter, the surface is referred to as the living body surface).

The electronic auscultation system 1 may include a position acquiring section 3. The position acquiring section 3 is configured so as to acquire position information indicating the position of the chest piece 21 with respect to the living body surface.

The electronic auscultation system 1 may include a contact state acquiring section 4. The contact state acquiring section 4 is configured so as to acquire contact state information indicating a contact state of the chest piece 21 with respect to the living body surface. The term "contact state" means a state which is comprehensively identified by the attitude and contact pressure of the chest piece 21 with respect to the living body surface.

The electronic auscultation system 1 may include a recording section 5. The recording section 5 is configured so as to record the position information and the contact state information while associating them with each other.

Fig. 1(B) diagrammatically illustrates, as an example, a plurality of positions which are on the living body surface 6, and at which the chest piece 21 is to be in contact with the living body surface during auscultation of heart sounds. The letter A indicates the left ventricular region, the letter B indicates the right ventricular region, the letter C indicates the pulmonary artery region, and the letter D indicates the aortic region.

When the user performs auscultation on the left ventricular region A by using the electronic stethoscope 2, the position acquiring section 3 acquires position information P1 indicating the position of the chest piece 21, and the contact state acquiring section 4 acquires contact state information C1 indicating the contact state of the chest piece 21. When the user performs auscultation on the right ventricular region B, the pulmonary artery region C, and the aortic region D by using the electronic stethoscope 2, similarly, position information P2 and contact state information C2 are acquired with respect to the right ventricular region B, position information P3 and contact state information C3 are acquired with respect to the pulmonary artery region C, and position information P4 and contact state information C4 are acquired with respect to the aortic region D.

That is, the recording section 5 records the position information P1 and the contact state information C1 while associating them with each other. Similarly, the recording section 5 records the position information P2 and the contact state information C2 while associating them with each other, the position information P3 and the contact state information C3 while associating them with each other, and the position information P4 and the contact state information C4 while associating them with each other.

The recording section 5 may be realized by at least one of a memory device, a hard disk drive, and a portable recording medium.

According to the configuration, during auscultation with the electronic stethoscope 2, it is possible to acquire not only biological information of the patient, but also information relating to the positon and contact state of the chest piece 21 with respect to the living body surface 6. For example, the way of auscultation by a skilled doctor can be acquired as objective data, and that of auscultation by a doctor in training can be objectively evaluated. When the data are used in the next and subsequent diagnosis, moreover, auscultation conditions for adequately acquiring biological information can be correctly reproduced regardless of a person who handles the stethoscope.

As illustrated in Fig. 1(A), specifically, the chest piece 21 may include a contact state sensor 22. The contact state sensor 22 includes at least one of an acceleration sensor, an inclination sensor, and a pressure sensor. The contact state acquiring section 4 is configured so as to acquire at least one of the attitude of the chest piece 21 and the contact pressure against the living body surface 6 as the contact state information based on an output from the contact state sensor 22.

In the case where the chest piece 21 includes an acceleration sensor, the acceleration sensor may be used for acquiring the position information of the chest piece 21.

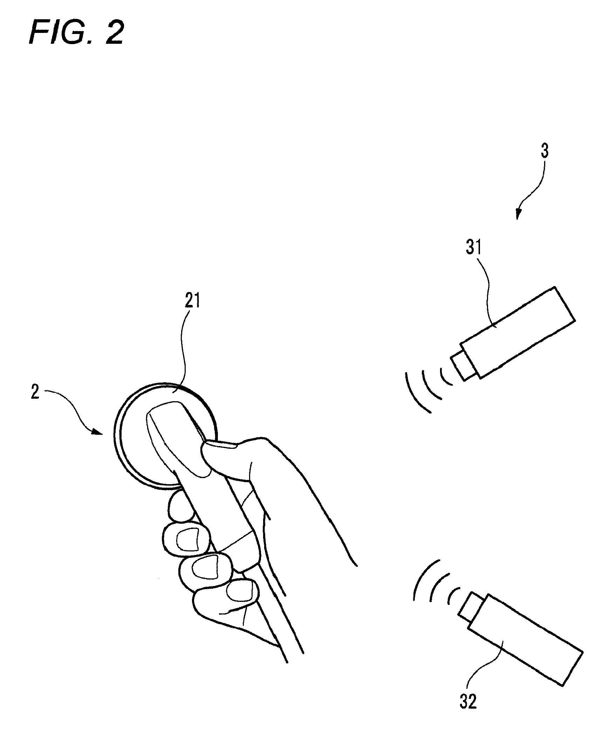

Fig. 2 illustrates an example of the position acquiring section 3. In the example, the position acquiring section 3 includes a wave transmitter 31 and a wave receiver 32. The wave transmitter 31 is configured so as to output a wave (such as an infrared ray or an ultrasonic wave) having a predetermined wavelength, toward the chest piece 21. The wave receiver 32 is configured so as to detect the wave which is output from the wave transmitter 31 and reflected by the chest piece 21. Here, the term "wave" includes both a transversal wave such as an electromagnetic wave, and a longitudinal wave such as a sound wave.

In this case, the position acquiring section 3 receives a signal output from the wave receiver 32 through wired or wireless communication, and processes the signal to acquire the position information. The kinds of the wave transmitter 31 and the wave receiver 32 may be appropriately determined as far as the position of the chest piece 21 can be known. The number and placements of the wave transmitter 31 and the wave receiver 32 may be adequately determined in accordance with the kinds of the wave transmitter 31 and the wave receiver 32.

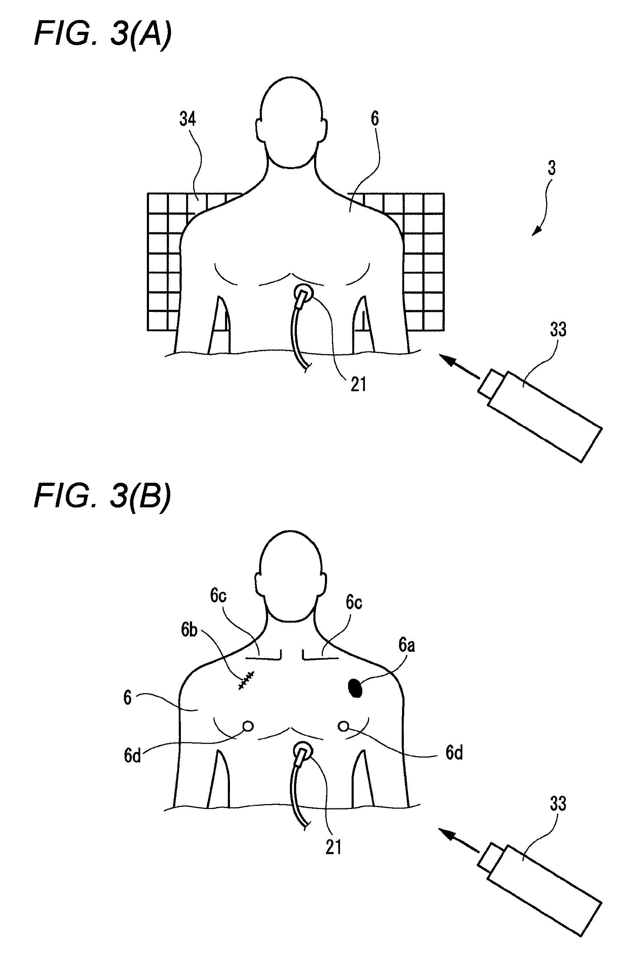

Fig. 3(A) illustrates another example of the position acquiring section 3. In the example, the position acquiring section 3 includes an imaging section 33 and a scale 34. The imaging section 33 is configured so as to take an image of the chest piece 21 and the scale 34 by using a predetermined wavelength band (the visible light band, the infrared band, or the like). The scale 34 has normalized scale marks. Here, the term "normalized" means that one unit (here, one scale mark) in the scale 34 is corresponded to another unit such as 1 cm or 1 inch.

Although, in the illustrated example, the scale 34 is placed behind the patient, the scale may be placed at a position adjacent to the auscultation position in the front half body of the patient. Alternatively, the scale may be projected to the front half body of the patient by a projector or the like.

In this case, the position acquiring section 3 processes the image taken by the imaging section 33 to acquire the position information of the chest piece 21. Specifically, the position acquiring section is configured so as to extract feature points of the image taken by the imaging section 33, and acquire the position information based on the feature points. In the example, the feature points are the scale marks of the scale 34.

As illustrated in Fig. 3 (B), the scale 34 may not be used. In this case, the imaging section 33 is configured so as to extract feature points of the living body surface 6 from the taken image. Examples of feature points of the living body surface 6 are a lentigo 6a, a scar 6b, clavicles 6c, and nipples 6d.

The position acquiring section 3 can be configured so as to acquire the temporal change of the position information. In this case, the recording section 5 is configured so as to record the temporal change of the position information.

According to the configuration, the movement path of the chest piece 21 during transfer auscultation can be recorded as objective data. When the data are used in the next and subsequent diagnosis, therefore, transfer auscultation can be correctly reproduced regardless of a person who handles the stethoscope.

The recording section 5 may be configured so as to record the timing when position information and contact state information are acquired, while associating the timing with the position information and the contact state information.

According to the configuration, analysis can be performed more multidirectionally, as in the case where, for example, auscultation data which are acquired at different times and dates of diagnoses are compared to each other.

As illustrated in Fig. 1 (A), the electronic auscultation system 1 may include a guidance information providing section 7. The guidance information providing section 7 is configured so as to, based on the position information recorded in the recording section 5, provide guidance information for guiding the chest piece 21 to the position indicated by the position information, to the user.

According to the configuration, the position at which the chest piece 21 is in contact with the patient can be made constant in a plurality of opportunities regardless of a person who handles the stethoscope. It is highly probable that such a position has been determined as a position where biological information can be correctly acquired. Therefore, the reliability of diagnosis which is performed in a plurality of opportunities can be enhanced. The stethoscope which is guided by the guidance information is not always necessary to be the electronic stethoscope 2.

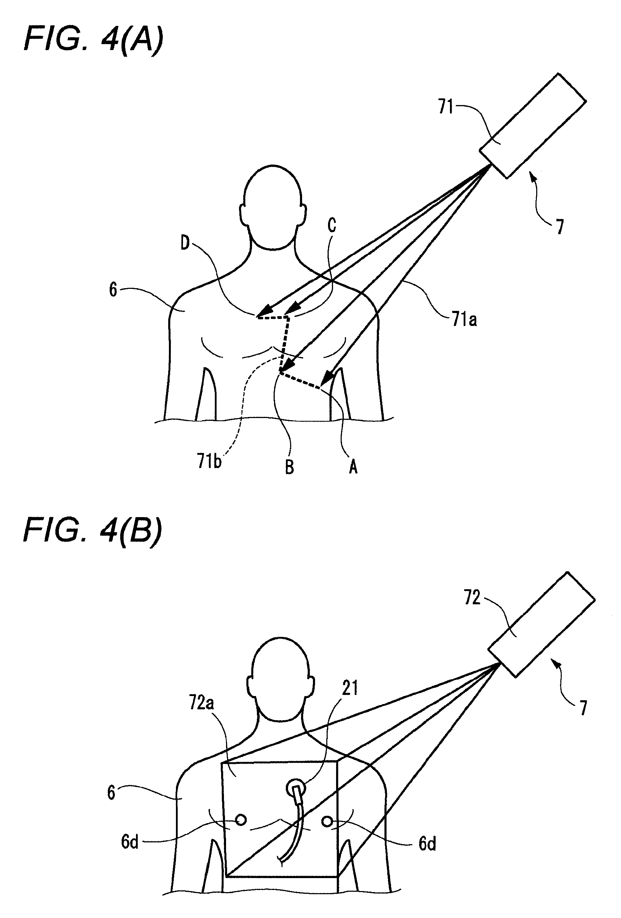

Fig. 4(A) shows an example of the guidance information providing section 7. In the example, the guidance information providing section 7 may include a pointer 71. The pointer 71 may include a light source which can emit a light beam 71a. The kind of the light source is not limited as far as it can emit light beams with a certain high level of directionality. The pointer 71 outputs the light beam 71a indicating the left ventricular region A, right ventricular region B, pulmonary artery region C, and aortic region D which are auscultation positions, as guidance information based on the sets of position information P1 to P4 recorded in the recording section 5. Namely, the guidance information is optically indicated on the living body surface 6. The plurality of auscultation positions may be sequentially indicated one by one in accordance with a predetermined auscultation sequence, or collectively indicated at one time by a plurality of light beams.

According to the configuration, the user can correctly place the chest piece at predetermined positions while being guided by the light beam 71a. Therefore, the position at which the chest piece is in contact with the living body can be made constant in a plurality of opportunities regardless of a person who handles the stethoscope.

In the case where the recording section 5 is configured so as to record also the temporal change of the position information, the pointer 71 may be configured so that a light beam 71b which is indicated by the broken line in the figure instructs the movement path of the chest piece. In this case, the user can correctly move the chest piece along the predetermined path while being guided by the light beam 71b. Therefore, the movement path in transfer auscultation can be made constant in a plurality of opportunities regardless of a person who handles the stethoscope.

In the case of transfer auscultation, also the movement velocity of the chest piece is contained in the auscultation conditions to be reproduced. In the configuration where the light beam 71b is moved at a predetermined velocity, the user can correctly move the chest piece along the predetermined path while being guided by the light beam 71b. Therefore, conditions of transfer auscultation can be reproduced in a plurality of opportunities regardless of a person who handles the stethoscope.

Fig. 4(B) shows another example of the guidance information providing section 7. In the example, the guidance information providing section 7 includes a projector 72. The projector 72 is configured so as to project an image 72a as guidance information onto the living body surface 6 of the patient. Namely, the guidance information is optically indicated on the living body surface 6. The image 72a contains auscultation practice which was performed on the patient in the past. Examples of the image 72a are an image which is taken by the imaging section 33 illustrated in Figs. 3(A) and 3(B), and a computer graphics image which is modeled based on the taken image and the position information acquired by the wave transmitter 31 and wave receiver 32 that are illustrated in Fig. 2.

In the illustrated example, in the image 72a, the nipples 6d functioning as an example of feature points can be identified. The user can determine the position of the patient with reference to the feature points. In the image 72a, also the position of the chest piece 21 in auscultation which was performed in the past can be identified. The user can place the chest piece of the stethoscope so that the chest piece overlaps with the image of the chest piece 21 that is projected onto the living body surface 6 of the patient whose position is determined. The stethoscope which is guided by the guidance information is not always necessary to be the electronic stethoscope 2.

According to the configuration, the user can correctly place the chest piece 21 at a predetermined position while checking the position of the chest piece which was placed in the past auscultation. Therefore, the position at which the chest piece 21 is in contact with the living body can be made constant in a plurality of opportunities regardless of a person who handles the stethoscope.

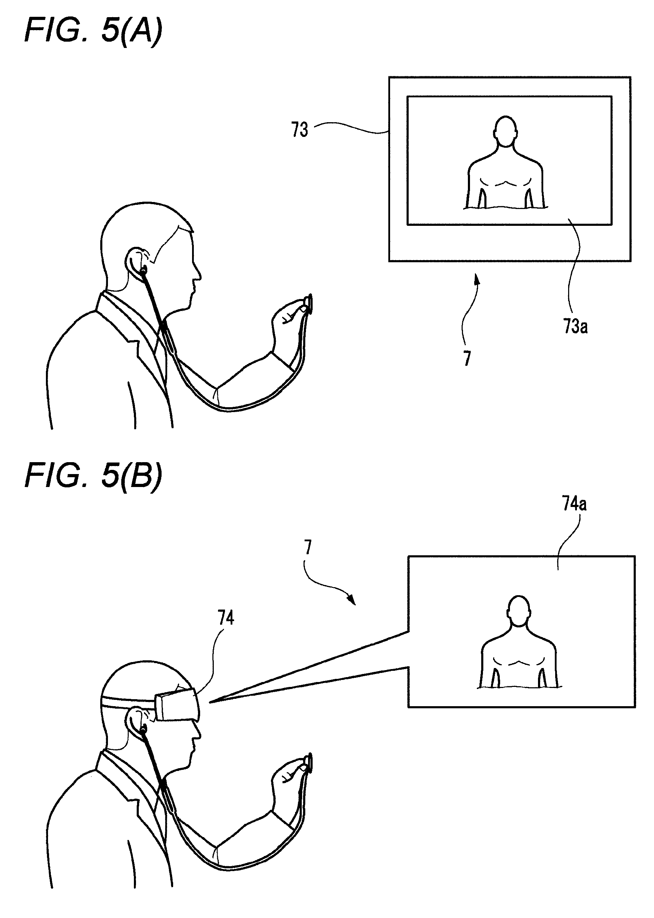

Fig. 5 (A) shows a further example of the guidance information providing section 7. In the example, the guidance information providing section 7 includes a displaying section 73. The displaying section 73 is configured so as to display an image 73a as guidance information. The image 73a is an image corresponding to the image 72a which is illustrated in Fig. 4(B). The displaying section 73 may be a related-art monitor or the like.

According to the configuration, the user can correctly place the chest piece 21 at a predetermined position while referring to the position at which the chest piece was placed in auscultation that was performed in the past. Therefore, the position at which the chest piece 21 is in contact with the living body can be made constant in a plurality of opportunities regardless of a person who handles the stethoscope. Moreover, the existing facility can be used, and therefore the electronic auscultation system 1 can be economically configured. The stethoscope which is guided by the guidance information is not always necessary to be the electronic stethoscope 2.

Fig. 5(B) shows a further example of the guidance information providing section 7. In the example, the guidance information providing section 7 includes a head mount display 74. The head mount display 74 is configured so as to display an image 74a as guidance information. The image 74a is an image corresponding to the image 72a which is illustrated in Fig. 4(B), and displayed superimposedly on an image (whether a real image or a taken image) of the patient which is displayed on the head mount display 74. The user wearing the head mount display 74 can determine the position of the patient with reference to the feature point which is displayed in the image 74a, and place the chest piece of the stethoscope so as to overlap with the image of the chest piece 21 contained in the image 74a that is displayed so as to overlap with the patient whose position has been determined. The stethoscope which is guided by the guidance information is not always necessary to be the electronic stethoscope 2.

According to the configuration, the user can correctly place the chest piece at a predetermined position while checking the position of the chest piece 21 which was placed in the past auscultation. Therefore, the position at which the chest piece is in contact with the living body can be made constant in a plurality of opportunities regardless of a person who handles the stethoscope.

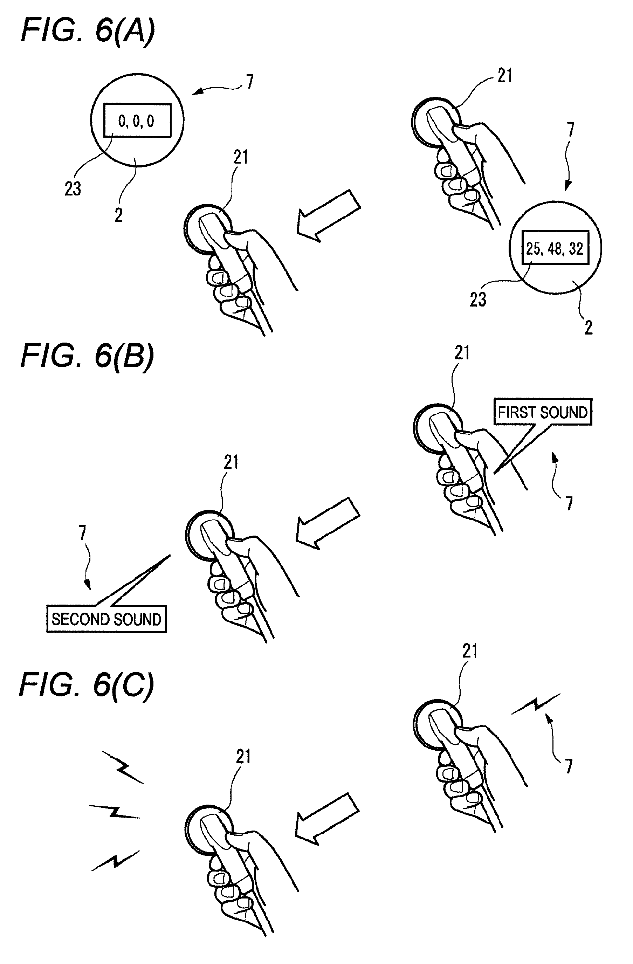

Fig. 6 (A) shows a further example of the guidance information providing section 7. In the example, the guidance information providing section 7 includes a displaying section 23 disposed in the electronic stethoscope 2. The displaying section 23 is configured so as to display, as guidance information, a value indicating the distance to a position which is indicated by the position information recorded in the recording section 5. For example, the guidance information providing section 7 acquires the difference between three-dimensional coordinates (the auscultation position) indicated by the position information recorded in the recording section 5 and those indicating the current position of the chest piece 21, and the result of the acquisition is displayed on the displaying section 23. The current position of the chest piece 21 is acquired by the position acquiring section 3. When the chest piece 21 is placed at the predetermined auscultation position, the difference between three-dimensional coordinates indicating the auscultation position and those indicating the current position is zero. Because of the fact that the value displayed on the displaying section 23 is zero, the user can know that the chest piece 21 is placed at the predetermined position.

According to the configuration, the user can correctly place the chest piece 21 at the predetermined position while checking that the value displayed on the displaying section 23 approaches to zero. Therefore, the position at which the chest piece is in contact with the living body can be made constant in a plurality of opportunities regardless of a person who handles the stethoscope.

The value indicating the difference between the auscultation position and the current position of the chest piece 21 may be displayed in the image 74a displayed on the head mount display 74 illustrated in Fig. 5(B), in place of the display on the displaying section 23.

Fig. 6(B) shows a further example of the guidance information providing section 7. In the example, the guidance information providing section 7 is configured so as to output sounds which change in accordance with the distance between the position information indicating the auscultation position recorded in the recording section 5 and the current position of the chest piece 21. The sounds may be output from the electronic stethoscope 2, or another sound source.

In the illustrated example, when the chest piece 21 is at a position which is remote from the predetermined auscultation position, first sound is output, and, when the chest piece 21 is placed at the predetermined auscultation position, second sound is output. An example of the difference between the first sound and the second sound is the sound volume. When the chest piece 21 becomes closer to the predetermined auscultation position, for example, the volume of the output sound is made larger.

Another example of the difference between the first sound and the second sound is the sound frequency (pitch). When the chest piece 21 becomes closer to the predetermined auscultation position, for example, the pitch (frequency) of the output sound is higher. A further example of the difference between the first sound and the second sound is the interval pitch of sounds which are repeatedly output. When the chest piece 21 becomes closer to the predetermined auscultation position, for example, the interval pitch of sounds which are repeatedly output is shorter. The first sound may be silent sound, and the second sound may be predetermined sound. In this case, only when the chest piece 21 is placed at the predetermined auscultation position, the predetermined sound is output. The predetermined sound may be a voice announcement. The relationships of the volume levels, frequency levels, and lengths of the interval pitches of the sound may be reversed to those of the above-described example.

According to the configurations, the user can correctly place the chest piece 21 at the predetermined position while being guided by the output sound. Therefore, the position at which the chest piece is in contact with the living body can be made constant in a plurality of opportunities regardless of a person who handles the stethoscope.

Fig. 6(C) shows a further example of the guidance information providing section 7. In the example, the guidance information providing section 7 is configured so as to output vibrations which change in accordance with the distance between the position information indicating the auscultation position recorded in the recording section 5 and the current position of the chest piece 21.

In the illustrated example, when the chest piece 21 is at a position which is remote from the predetermined auscultation position, a first vibration is output, and, when the chest piece 21 is placed at the predetermined auscultation position, a second vibration is output. An example of the difference between the first vibration and the second vibration is the vibration level. When the chest piece 21 becomes closer to the predetermined auscultation position, for example, the level of the output vibration is made higher.

Another example of the difference between the first vibration and the second vibration is the pitch of vibrations which are repeatedly output. When the chest piece 21 becomes closer to the predetermined auscultation position, for example, the pitch of vibrations which are repeatedly output is made higher. The first vibration may be non-vibration, and the second vibration may be predetermined vibration. In this case, only when the chest piece 21 is placed at the predetermined auscultation position, the predetermined vibration is output. The relationships of the levels of the vibrations and lengths of the pitches may be reversed to those of the above-described example.

In a further example of the guidance information providing section 7, although not illustrated, the electronic stethoscope 2 includes a light emitter. In this case, the guidance information providing section 7 is configured so as to output light beams which change in accordance with the distance between the position information indicating the auscultation position recorded in the recording section 5 and the current position of the chest piece 21. For example, when the chest piece 21 is at a position which is remote from the predetermined auscultation position, a first light beam is emitted, and, when the chest piece 21 is placed at the predetermined auscultation position, a second light beam is emitted. An example of the difference between the first and second light beams is the luminance. When the chest piece 21 becomes closer to the predetermined auscultation position, for example, the luminance of the emitted light beam is made higher.

Another example of the difference between the first and second light beams is the color of the light beam. When the chest piece 21 becomes closer to the predetermined auscultation position, for example, the color of the emitted light beam is changed from green to red. A further example of the difference between the first and second light beams is the interval of blinking. When the chest piece 21 becomes closer to the predetermined auscultation position, for example, the interval of blinking is made shorter. The first light beam may be no-light emission, and the second light beam may be a predetermined light beam. In this case, only when the chest piece 21 is placed at the predetermined auscultation position, the predetermined light beam is emitted. The relationships of the luminance levels, colors, and lengths of the blinking intervals may be reversed to those of the above-described example.

According to the configuration, the user can correctly place the chest piece 21 at the predetermined position while being guided by the emitted light beam. Therefore, the position at which the chest piece is in contact with the living body can be made constant in a plurality of opportunities regardless of a person who handles the stethoscope.

The examples (value, sound, vibration, and light beam) of the guidance information which changes in accordance with the distance to the auscultation position have been described with reference to Figs. 6(A) to 6(C). Two or more of the examples may be arbitrarily combined to each other.

According to the configurations, the user can correctly place the chest piece 21 at the predetermined position while being guided by the output sound. Therefore, the position at which the chest piece is in contact with the living body can be made constant in a plurality of opportunities regardless of a person who handles the stethoscope.

As illustrated in Fig. 1 (A), the electronic auscultation system 1 may further include a reproduction information providing section 8. The reproduction information providing section 8 is configured so as to, based on the contact state information recorded in the recording section 5, provide reproduction information for reproducing the contact state of the chest piece 21 indicated by the contact state information, to the user.

According to the configuration, the contact state of the chest piece 21 with respect to the living body surface 6 of the patient can be made constant in a plurality of opportunities regardless of a person who handles the stethoscope. When not only the placement of the chest piece 21, but also the contact state is reproduced by using objective data, reproduction of auscultation conditions, which is said to require skills can be easily realized. Therefore, the reliability of diagnosis which is performed in a plurality of opportunities can be enhanced.

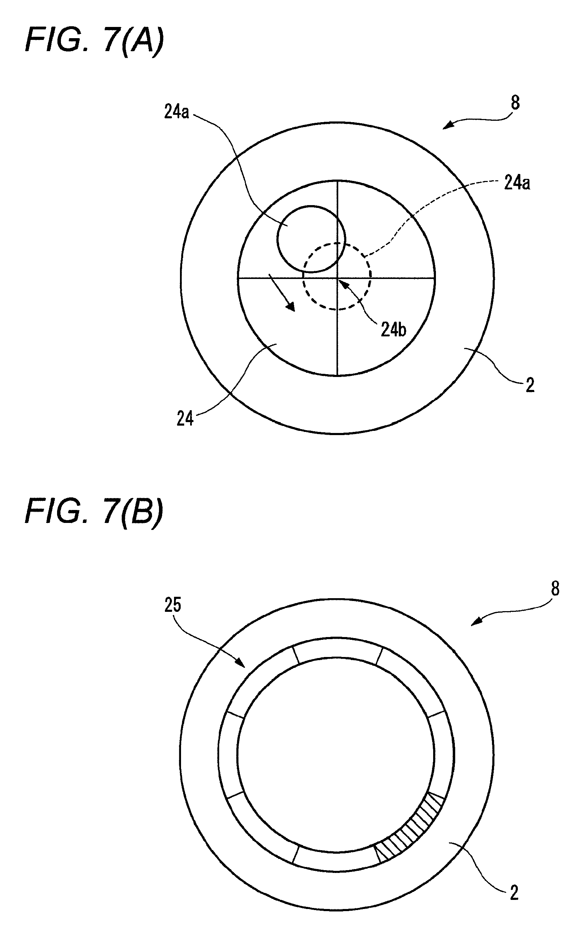

Fig. 7(A) illustrates an example of the reproduction information providing section 8. In the example, the reproduction information providing section 8 includes a displaying section 24 disposed in the electronic stethoscope 2. The displaying section 24 is configured so that a circle 24a corresponding to the current posture of the chest piece 21 is displayed, and, when the circle 24a is positioned at the center 24b of the cross reticle, it is indicated that the chest piece 21 attains a target posture. In the illustrated example, the circle 24a is displayed on the upper left side of the center 24b. In this case, when the chest piece 21 is inclined toward the direction corresponding to the right lower side in the figure, the circle 24a is moved to the center 24b.

In the case where information relating to the contact pressure is contained in the reproduction information, the displaying section 24 may be configured so that, when the contact pressure reaches the target pressure, the color of the circle 24a is changed. The user adjusts the position of the circle 24a as described above to change the posture of the chest piece 21, and causes the color of the circle 24a positioned at the circle 24a to be changed, thereby obtaining the target contact pressure.

According to the configuration, the contact state of the chest piece 21 corresponding to the contact state information recorded in the recording section 5 can be reproduced through intuitive operations. Therefore, the contact state of the chest piece 21 with respect to the living body surface 6 of the patient can be easily made constant in a plurality of opportunities regardless of a person who handles the stethoscope.

Fig. 7 (B) illustrates another example of the reproduction information providing section 8. In the example, the reproduction information providing section 8 includes a displaying section 25 disposed in the electronic stethoscope 2. The displaying section 25 may include, for example, eight light emitters, and is configured so that one of the light emitters that is positioned in the direction along which the chest piece 21 must be inclined to attained the target posture emits light. In the illustrated example, the light emitter that is positioned in the direction corresponding to the right lower side in the figure emits light. In this case, the user inclines the chest piece 21 in the direction corresponding to the right lower side in the figure, thereby causing the chest piece 21 to attain the target posture.

In the case where information relating to the contact pressure is contained in the reproduction information, the displaying section 25 may be configured so that, when the contact pressure reaches the target pressure, the color of the light emitter is changed. The user changes the posture of the chest piece 21 in accordance with the above-described guidance of the light emitter, to change the color of the light emitter, thereby attaining the target contact pressure.

According to the configuration, the contact state of the chest piece 21 corresponding to the contact state information recorded in the recording section 5 can be reproduced through intuitive operations. Therefore, the contact state of the chest piece 21 with respect to the living body surface 6 of the patient can be easily made constant in a plurality of opportunities regardless of a person who handles the stethoscope.

The display indicating the difference between the current toughing state of the chest piece 21 and the target toughing state may be displayed in the image 74a displayed on the head mount display 74 illustrated in Fig. 5(B), in place of the display on the displaying section 24 or 25.

The difference between the current toughing state of the chest piece 21 and the target toughing state is not always required to be visually provided to the user. For example, the difference may be acoustically provided to the user through voice guidance. In the examples illustrated in Figs. 7 (A) and 7 (B), the reproduction information providing section 8 outputs a message "Incline the chest piece toward the right lower side, and press the chest piece slightly strongly." The position of the sound source may be adequately determined.

The electronic auscultation system 1 of the embodiment is configured on the premise that the opportunity when the position information and the contact state information are acquired is different from that when the guidance information and the reproduction information are provided. The user who uses the electronic stethoscope 2 relating to the acquisition of the position information and the contact state information may be identical to or different from the user who is provided with the guidance information and the reproduction information. Moreover, the place where the position information and the contact state information are acquired may be identical to or different from that where the guidance information and the reproduction information are provided.

In the embodiment, at least a part of the functions of the position acquiring section 3, the contact state acquiring section 4, the guidance information providing section 7, and the reproduction information providing section 8 is realized by software which is executed in cooperation of a processor and memory that are communicably connected to each other. Examples of the processor are a CPU and an MPU. Examples of the memory are a RAM and a ROM. At least two of the position acquiring section 3, the contact state acquiring section 4, the guidance information providing section 7, and the reproduction information providing section 8 can be realized by a common processor and a common memory.

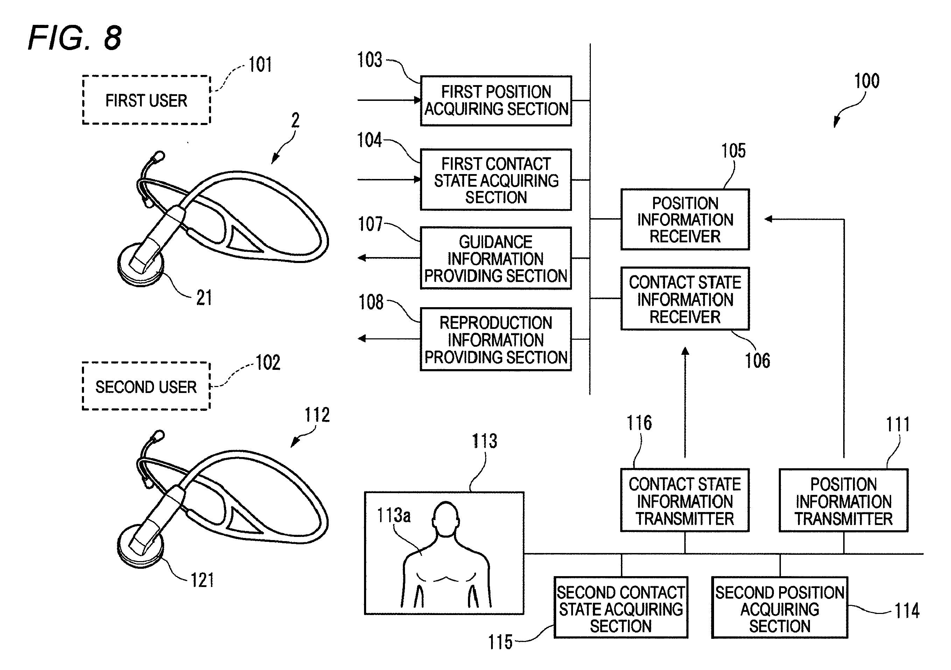

Fig. 8 diagrammatically illustrates the functional configuration of an electronic auscultation system 100 of a second embodiment. The components identical or equivalent to those of the electronic auscultation system 1 of the first embodiment are denoted by the same reference numerals, and repeated description will be omitted.

The electronic auscultation system 100 may include the electronic stethoscope 2. The electronic stethoscope 2 is assumed to be used by a first user 101. The electronic stethoscope 2 may include the chest piece 21 which is caused to be in contact with the living body surface 6.

The electronic auscultation system 100 may include a first position acquiring section 103. The first position acquiring section 103 is configured so as to acquire first position information indicating the position of the chest piece 21 with respect to the living body surface 6. As the configuration for acquiring the first position information, the configurations which are exemplified with respect to the position acquiring section 3 of the electronic auscultation system 1 of the first embodiment may be employed.

The electronic auscultation system 100 may include a position information transmitter 111. The position information transmitter 111 is placed in a place which is remote from the first position acquiring section 103. The position information transmitter 111 is configured so as to transmit target position information indicating a target position in the living body surface 6 where the chest piece 21 is to be placed.

The electronic auscultation system 100 may include a position information receiver 105. The position information receiver 105 is placed in a place which is remote from the position information transmitter 111. The position information receiver 105 is configured so as to receive the target position information transmitted from the position information transmitter 111.

The electronic auscultation system 100 may include a guidance information providing section 107. The guidance information providing section 107 is configured so as to, based on the first position information acquired by the first position acquiring section 103, and the target position information received by the position information receiver 105, provide guidance information for guiding the chest piece 21 to the target position indicated by the target position information, to the first user 101. As the configuration for providing the guidance information, the configurations which are exemplified with respect to the guidance information providing section 7 of the electronic auscultation system 1 of the first embodiment may be employed.

For example, the target position information may be transmitted by a second user 102. The second user 102 is in a place which is remote from the first user 101. For example, the second user 102 is a doctor. Examples of the first user 101 are a doctor who is in a room different from that where the second user 102 stays, a doctor who is in a hospital different from that where the second user 102 stays, and a family member of the patient in home medical care.

According to the configuration, the first user 101 can correctly place the chest piece 21 at a predetermined position based on the target position information which is instructions provided from a remote place. Therefore, the first user 101 can perform auscultation based on the instructions provided from the remote place. Consequently, auscultation conditions for adequately acquiring biological information can be correctly reproduced regardless of a person who handles the stethoscope.

The electronic auscultation system 100 may include a dummy stethoscope 112. The dummy stethoscope 112 is assumed to be used by the second user 102. The dummy stethoscope 112 may include a dummy chest piece 121.

The electronic auscultation system 100 may include a displaying device 113. The displaying device 113 is configured so as to display an image containing a dummy living body surface 113a corresponding to the living body surface 6. Examples of the image containing the dummy living body surface 113a are an image which is taken by an adequate imaging device, and a computer graphics image which is modeled based on the taken image and the like. The image may be an image which is obtained by imaging in real time the patient facing the first user 101, or that which was taken in past auscultation of the patient.

The electronic auscultation system 100 may include a second position acquiring section 114. The second user 102 places the dummy chest piece 121 at an adequate auscultation position in the dummy living body surface 113a displayed on the displaying device 113. The second position acquiring section 114 is configured so as to acquire second position information indicating the position of the dummy chest piece 121 with respect to the dummy living body surface 113a.

As the configuration for acquiring the second position information, the configurations which are exemplified with respect to the position acquiring section 3 of the electronic auscultation system 1 of the first embodiment may be employed. Alternatively, the second position information may be acquired by using the displaying device 113 having a touch panel screen, and detecting a position where the dummy chest piece 121 is in contact with the touch panel screen.

In this case, the position information transmitter 111 is configured so as to transmit the second position information acquired by the second position acquiring section 114, as the target position information.

According to the configuration, the second user 102 can transmit the target position information to the first user 101 through more intuitive operations. Therefore, the accuracy of the target position information can be enhanced, and the first user 101 can place more correctly the chest piece 21 at the predetermined position. Consequently, auscultation conditions for adequately acquiring biological information can be more correctly reproduced regardless of a person who handles the stethoscope.

The electronic auscultation system 100 may include a first contact state acquiring section 104. The first contact state acquiring section 104 is configured so as to acquire first contact state information indicating a contact state of the chest piece 21 of the electronic stethoscope 2 with respect to the living body surface 6. As the configuration for acquiring the first contact state information, the configurations which are exemplified with respect to the contact state acquiring section 4 of the electronic auscultation system 1 of the first embodiment may be employed.

The electronic auscultation system 100 may include a second contact state acquiring section 115. The second contact state acquiring section 115 is configured so as to acquire second contact state information indicating a contact state of the dummy chest piece 121 of the dummy stethoscope 112 with respect to the dummy living body surface 113a.

As the configuration for acquiring the second contact state information, the configurations which are exemplified with respect to the contact state acquiring section 4 of the electronic auscultation system 1 of the first embodiment may be employed. Alternatively, the second contact state information may be acquired by using the displaying device 113 having a touch panel screen, and detecting a contact pressure of the dummy chest piece 121 with respect to the touch panel screen.

The electronic auscultation system 100 may include a contact state information transmitter 116. The contact state information transmitter 116 is placed in a place which is remote from the first contact state acquiring section 104. The contact state information transmitter 116 is configured so as to transmit the second contact state information acquired by the second contact state acquiring section 115.

The electronic auscultation system 100 may include a contact state information receiver 106. The contact state information receiver 106 is placed in a place which is remote from the contact state information transmitter 116. The contact state information receiver 106 is configured so as to receive the second contact state information transmitted from the contact state information transmitter 116.

The electronic auscultation system 100 may include a reproduction information providing section 108. The reproduction information providing section 108 is configured so as to, based on the first contact state information acquired by the first contact state acquiring section 104, and the second contact state information received by the contact state information receiver 106, provide reproduction information for reproducing the contact state of the dummy chest piece 121 indicated by the second contact state information, to the first user 101. As the configuration for providing the reproduction information, the configurations which are exemplified with respect to the reproduction information providing section 8 of the electronic auscultation system 1 of the first embodiment may be employed.

According to the configuration, the second user 102 can transmit the information relating to the contact state of the chest piece 21 to the first user 101 through intuitive operations. When not only the placement of the chest piece 21, but also the contact state is reproduced by using objective data, the first user 101 can easily realize reproduction of auscultation conditions, which is said to require skills. Therefore, auscultation conditions for adequately acquiring biological information can be more correctly reproduced regardless of a person who handles the stethoscope.

The second position acquiring section 114 may be configured so as to acquire second position information indicating the sight line position of the second user 102 with respect to the dummy living body surface 113a. A related-art technique for detecting the sight line is used in the detection of the sight line position of the second user 102. In this case, the position information transmitter 111 transmits the thus acquired second position information as the target position information.

According to the configuration, even when the second user 102 does not hold the stethoscope, the second user can transmit the target position information to the first user 101.

In the embodiment, at least a part of the functions of the first position acquiring section 103, the first contact state acquiring section 104, the position information receiver 105, the contact state information receiver 106, the guidance information providing section 107, and the reproduction information providing section 108 is realized by software which is executed in cooperation of a processor and memory that are communicably connected to each other. Examples of the processor are a CPU and an MPU. Examples of the memory are a RAM and a ROM. At least two of the first position acquiring section 103, the first contact state acquiring section 104, the position information receiver 105, the contact state information receiver 106, the guidance information providing section 107, and the reproduction information providing section 108 can be realized by a common processor and a common memory.

In the embodiment, at least a part of the functions of the position information transmitter 111, the second position acquiring section 114, the second contact state acquiring section 115, and the contact state information transmitter 116 is realized by software which is executed in cooperation of a processor and memory that are communicably connected to each other. Examples of the processor are a CPU and an MPU. Examples of the memory are a RAM and a ROM. At least two of the position information transmitter 111, the second position acquiring section 114, the second contact state acquiring section 115, and the contact state information transmitter 116 can be realized by a common processor and a common memory.

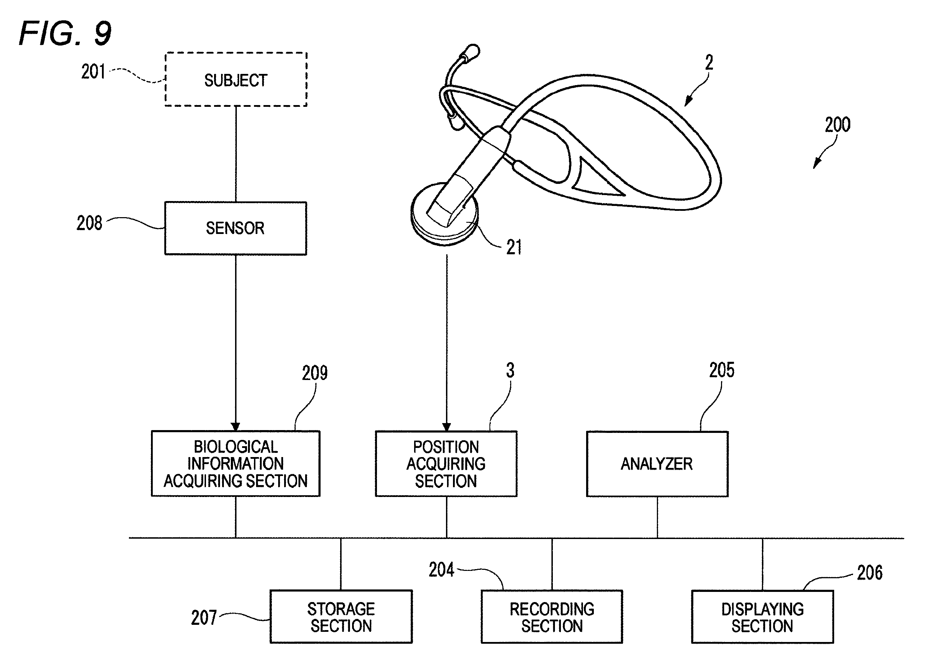

Fig. 9 diagrammatically illustrates the functional configuration of an electronic auscultation system 200 of a third embodiment. The components identical or equivalent to those of the electronic auscultation system 1 of the first embodiment are denoted by the same reference numerals, and repeated description will be omitted.

The electronic auscultation system 200 may include a recording section 204. The recording section 204 is configured so as to record biological sound of the subject 201 which is acquired through the chest piece 21, while associating the sound with the position information of the chest piece 21 which is acquired by the position acquiring section 3.

The recording section 204 may be realized by at least one of a memory device, a hard disk drive, and a portable recording medium.

The electronic auscultation system 200 may include an analyzer 205. The analyzer 205 is configured so as to analyze the biological sound, which is recorded in the recording section 204 while associating the sound with the position information. Examples of the analysis are amplitude analysis, frequency analysis, and cepstrum analysis.

The electronic auscultation system 200 may include a displaying section 206. The displaying section 206 is configured so as to display a result of analysis performed by the analyzer 205, while associating the result with the position information acquired by the position acquiring section 3.

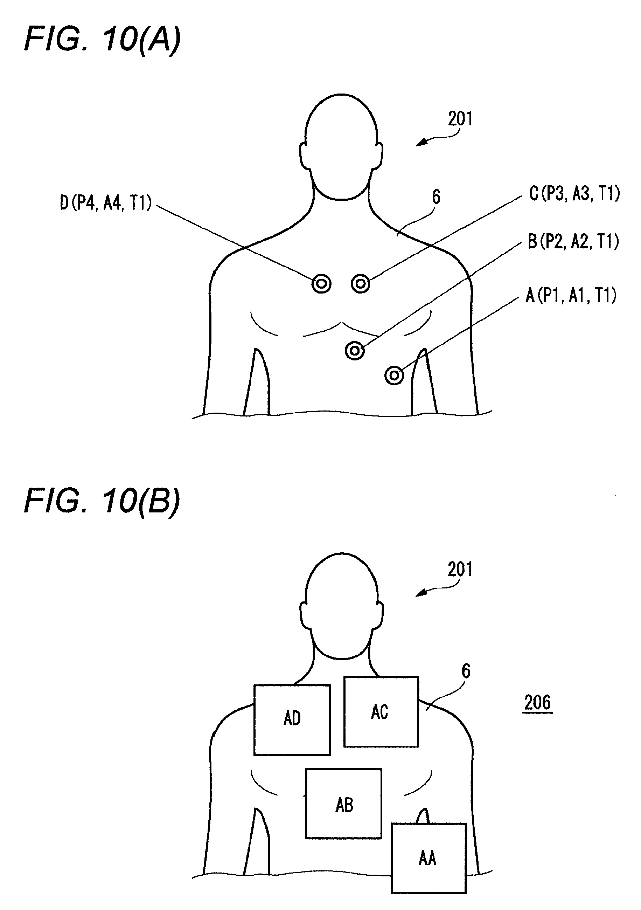

Fig. 10(A) diagrammatically illustrates relationships between data recorded in the recording section 204, and the auscultation position. Biological sound data A1 which are acquired in the left ventricular region A by the chest piece 21 are recorded while being associated with the position information P1 acquired by the position acquiring section 3. Similarly, biological sound data A2 which are acquired in the right ventricular region B are recorded while being associated with the position information P2, biological sound data A3 which are acquired in the pulmonary artery region C are recorded while being associated with the position information P3, and biological sound data A4 which are acquired in the aortic region D are recorded while being associated with the position information P4.

As shown in Fig. 10(B), when the auscultation position is designated by the user, a result of analysis of biological sound which is associated with position information corresponding to the auscultation position is displayed on the displaying section 206. When the left ventricular region A is designated as the auscultation position by the use, for example, an analysis result AA of the biological sound data A1 associated with the position information P1 is displayed on the displaying section 206. In the following description, an analysis result of the biological sound data A2 is indicated by AB, that of the biological sound data A3 is indicated by AC, and that of the biological sound data A4 is indicated by AD.

As illustrated in the figure, the analysis results AA to AD may be displayed so as to overlap with positions corresponding to auscultation positions in an image showing the body of the subject 201, respectively. If correspondence relationships with auscultation positions can be known, the results may be displayed at arbitrary positions on the displaying section 206 while using, for example, leading lines illustrated in Fig. 10 (A). At least one of the analysis results AA to AD may be individually displayed in accordance with the designation of the auscultation position by the user, or all of the analysis results may be collectively displayed.

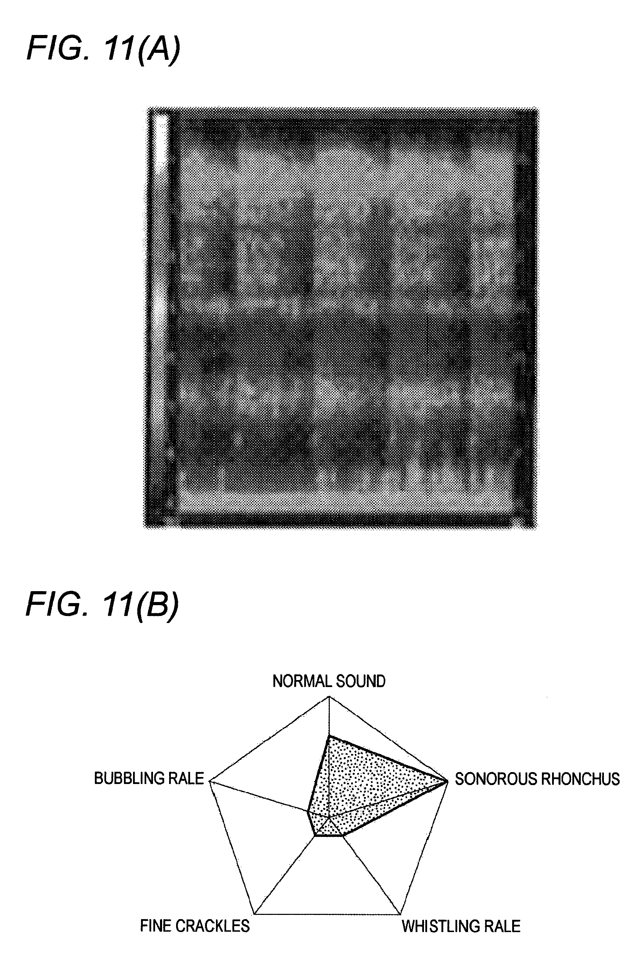

Examples of a displaying mode of the analysis results AA to AD are the current waveform of the biological sound, the average value of waveform amplitudes form a certain timing to another timing, the maximum and minimum values, the average, maximum, and minimum values of waveform amplitudes in the measurement time period, a spectrogram (see Fig. 11(A)) which is obtained as a result of analysis of the amplitude and the frequency, and a radar chart (see Fig. 11(B)).

According to the configuration, it is possible to easily manage the auscultation position where the result of analysis of biological sound which is acquired through the chest piece 21 is obtained. Consequently, diagnosis can be supported more correctly and multidirectionally.

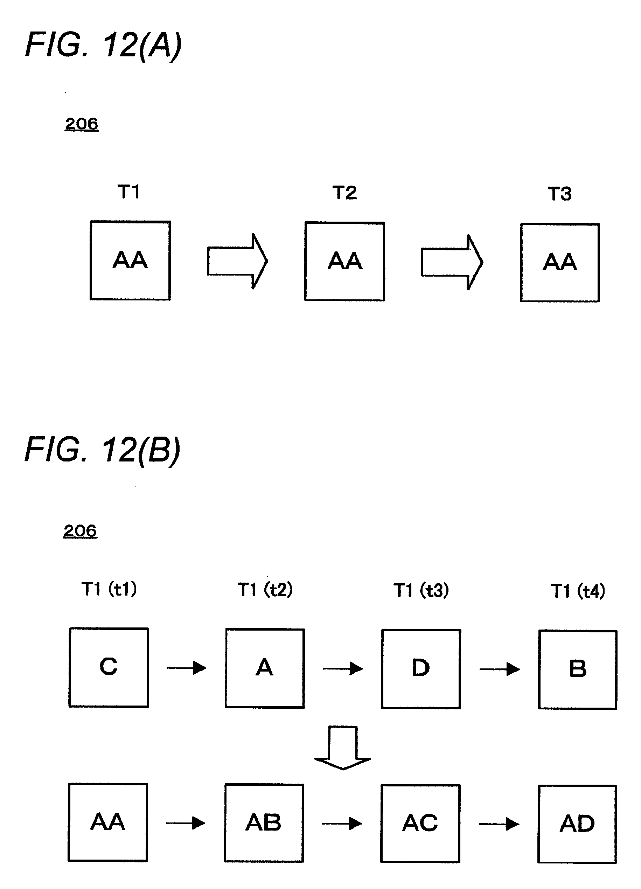

The recording section 204 is configured so as to record the timing when the position information and the biological sound are acquired, while associating the timing with the position information and the biological sound. Here, the term "timing" may mean different timings in one auscultation opportunity, or one auscultation opportunity may be deemed as the same "timing". In Fig. 10(A), one auscultation opportunity is indicated by a symbol T1. In Fig. 12 (B), different timings in the auscultation opportunity T1 are indicated by symbols t1 to t4, respectively.

In this case, the displaying section 206 is configured so as to display the analysis results obtained by the analyzer 205, in time sequence based on the above-described timing. In an example illustrated in Fig. 12(A), analysis results AA which were obtained respectively in a plurality of auscultation opportunities T1 to T3, and which relate to the left ventricular region A are displayed time-sequentially on the displaying section 206.

According to the configuration, it is possible to easily know the temporal change of biological sound which is acquired at a specific auscultation position. Consequently, diagnosis can be supported more correctly and multidirectionally.

As illustrated in Fig. 9, the electronic auscultation system 200 may include a storage section 207. The storage section 207 is configured so as to store a plurality of auscultation positions on the living body surface 6, and the auscultation sequence. In the case of auscultation of heart sounds, the storage section 207 stores a plurality of auscultation positions including the left ventricular region A, the right ventricular region B, the pulmonary artery region C, and the aortic region D. The storage section 207 further stores the auscultation sequence in which auscultation is to be performed in the sequence of the left ventricular region A, the right ventricular region B, the pulmonary artery region C, and the aortic region D. The auscultation sequence may be changed depending on the disease and the patient.

The recording section 207 may be realized by at least one of a memory device, a hard disk drive, and a portable recording medium.

In this case, the displaying section 206 is configured so as to display the results of analysis performed by the analyzer 205, correspondingly with a plurality of auscultation positions, based on the auscultation sequence stored in the storage section 207. Fig. 12(B) illustrates a case where the user performed auscultation in a wrong sequence. Specifically, it is illustrated that, in the auscultation opportunity T1, auscultation was performed in the sequence of the pulmonary artery region C (timing t1), the left ventricular region A (timing t2), the aortic region D (timing t3), and the right ventricular region B (timing t4).

According to the configuration of the embodiment, the auscultation sequence in which auscultation is to be performed in the sequence of the left ventricular region A, the right ventricular region B, the pulmonary artery region C, and the aortic region D is stored in the storage section 207, and therefore the displaying section 206 displays analysis results so that the analysis results are arranged in the sequence of the analysis result AA relating to the left ventricular region, a analysis result AB relating to the right ventricular region, a analysis result AC relating to the pulmonary artery region C, and a analysis result AD relating to the aortic region D.

According to the configuration, even in the case where a person who is unaccustomed to auscultation performs auscultation in a wrong procedure, the analysis result can be presented as if the auscultation is performed in a correct procedure. Consequently, diagnosis can be supported more correctly and multidirectionally.

As illustrated in Fig. 9, the electronic auscultation system 200 may include a sensor 208 and a biological information acquiring section 209. The sensor 208 is configured so as to be attached to the subject 201. Examples of the sensor 208 are a probe of a pulse photometer, electrodes of an electrocardiograph, those of an electroencephalograph, and a body temperature sensor. The biological information acquiring section 209 is configured so as to acquire biological information of the subject 201 through the sensor 208.

In this case, the displaying section 206 is configured so as to display the biological information acquired by the biological information acquiring section 209, and the analysis results acquired by the analyzer 205, in a temporally synchronized manner.

According to the configuration, the result of analysis of biological sound can be studied while referring to the biological information. In the case where an abnormality is found in the biological sound, for example, it is possible to check a change (s) which occurs in another kind(s) of biological information at the timing when the abnormality is found. Consequently, diagnosis can be supported more correctly and multidirectionally.

In the embodiment, at least a part of the functions of the position acquiring section 3, the analyzer 205, and the biological information acquiring section 209 is realized by software which is executed in cooperation of a processor and memory that are communicably connected to each other. Examples of the processor are a CPU and an MPU. Examples of the memory are a RAM and a ROM. At least two of the position acquiring section 3, the analyzer 205, and the biological information acquiring section 209 can be realized by a common processor and a common memory.

The foregoing description of the embodiments has been made in order to facilitate understanding of the presently disclosed subject matter, and is not intended to limit the presently disclosed subject matter. It is a matter of course that the presently disclosed subject matter may be changed or improved without departing the spirit thereof, and includes equivalents thereof.

Although, in the above-described embodiments, the case where heart sounds are auscultated has been exemplified, the configurations of the embodiments may be applied also to a case where respiratory sounds or abdominal sounds are auscultated.

The configuration of the electronic auscultation system 200 of the third embodiment may be appropriately combined with the electronic auscultation system 1 of the first embodiment, or the electronic auscultation system 100 of the second embodiment.

## Claims

1. An electronic auscultation system comprising:
an electronic stethoscope including a chest piece which is adapted to be in contact with a living body surface;
a position acquiring section which is configured to acquire position information indicating a position of the chest piece with respect to the living body surface;
a contact state acquiring section which is configured to acquire contact state information indicating a contact state of the chest piece with respect to the living body surface; and
a recording section which is configured to record the position information and the contact state information while associating the position information with the contact state information.

2. The electronic auscultation system according to claim 1, wherein
the chest piece includes a contact state sensor including at least one of an acceleration sensor, an inclination sensor, and a pressure sensor, and
the contact state acquiring section is configured to acquire, as the contact state information, at least one of an attitude of the chest piece and a contact pressure of the chest piece with respect to the living body surface, based on an output from the contact state sensor.

3. The electronic auscultation system according to claim 1 or 2, wherein
the position acquiring section includes:
a wave transmitter which is configured to output a wave having a predetermined wavelength, toward the chest piece; and
a wave receiver which is configured to detect the wave that is output from the wave transmitter and reflected by the chest piece, and
the position acquiring section is configured to acquire the position information based on an output of the wave receiver.

4. The electronic auscultation system according to claim 1 or 2, wherein
the position acquiring section includes an imaging section which is configured to acquire an image of the living body surface and the chest piece, and
the position acquiring section is configured to acquire the position information based on the image.

5. The electronic auscultation system according to claim 4, wherein
the position acquiring section is configured to extract a feature point of the image, and is configured to acquire the position information based on the feature point.

6. The electronic auscultation system according to any one of claims 1 to 5, wherein
the recording section is configured to record a timing when the position information and the contact state information are acquired, while associating the timing with the position information and the contact state information.

7. The electronic auscultation system according to any one of claims 1 to 6, further comprising:
a guidance information providing section which, based on the position information recorded in the recording section, is configured to provide guidance information for guiding the chest piece to the position indicated by the position information, to a user.

8. The electronic auscultation system according to claim 7, wherein
the guidance information providing section is configured to optically indicate the guidance information on the living body surface.

9. The electronic auscultation system according to claim 8, wherein
the guidance information contains an image of auscultation practice, the image being taken in a past.

10. The electronic auscultation system according to claim 7, wherein
the guidance information includes at least one of: a value which indicates a distance to the position; a sound which changes in accordance with a distance to the position; a vibration which changes in accordance with a distance to the position; and a light beam which changes in accordance with a distance to the position.

11. The electronic auscultation system according to any one of claims 7 to 10, further comprising:
a reproduction information providing section which, based on the contact state information, is configured to provide reproduction information for reproducing the contact state of the chest piece indicated by the contact state information, to the user.

12. An electronic auscultation system comprising:
an electronic stethoscope including a chest piece which is adapted to be in contact with a living body surface, by a first user;
a first position acquiring section which is configured to acquire first position information indicating a position of the chest piece with respect to the living body surface;
a position information transmitter which is placed in a place that is remote from the first position acquiring section, and which is configured to transmit target position information indicating a target position in the living body surface where the chest piece is to be placed;
a position information receiver which is configured to receive the target position information transmitted from the position information transmitter; and
a guidance information providing section which, based on the position information acquired by the first position acquiring section, and the target position information received by the position information receiver, is configured to provide guidance information for guiding the chest piece to the target position, to the first user.

13. The electronic auscultation system according to claim 12, further comprising:
a dummy stethoscope including a dummy chest piece which is used by a second user;
a displaying device which is configured to display an image containing a dummy living body surface that corresponds to the living body surface; and
a second position acquiring section which is configured to acquire second position information indicating a position of the dummy chest piece with respect to the dummy living body surface, wherein
the position information transmitter transmits the second position information as the target position information.

14. The electronic auscultation system according to claim 13, further comprising:
a first contact state acquiring section which is configured to acquire first contact state information indicating a contact state of the chest piece with respect the living body surface;
a second contact state acquiring section which is configured to acquire second contact state information indicating a contact state of the dummy chest piece with respect to the dummy living body surface;
a contact state information transmitter which is configured to transmit the second contact state information;
a contact state information receiver which is placed in a place that is remote from the contact state information transmitter, and which is configured to receive the second contact state information; and
a reproduction information providing section which, based on the first contact state information and the second contact state information, is configured to provide reproduction information for reproducing the contact state of the dummy chest piece indicated by the second contact state information, to the first user.

15. The electronic auscultation system according to claim 12, further comprising:
a displaying device which is configured to display an image containing a dummy living body surface that corresponds to the living body surface; and
a second position acquiring section which is configured to acquire second position information indicating a sight line position of the second user with respect to the dummy living body surface, wherein
the position information transmitter transmits the second position information as the target position information.

16. An electronic auscultation system comprising:
an electronic stethoscope including a chest piece which is to adapted to be in contact with a living body surface of a subject;
a position acquiring section which is configured to acquire position information indicating a position of the chest piece with respect to the living body surface;
a recording section which is configured to record biological sound that is acquired through the chest piece, while associating the sound with the position information;
an analyzer which is configured to analyze the biological sound that is recorded while being associated with the position information; and
a displaying section which is configured to display a result of analysis performed by the analyzer, while associating the result with the position information.

17. The electronic auscultation system according to claim 16, wherein
the recording section is configured to record a timing when the position information and the biological sound are acquired, while associating the timing with the position information and the biological sound, and
the displaying section is configured to display time-sequentially the result of analysis based on the timing.

18. The electronic auscultation system according to claim 16 or 17, further comprising:
a storage section which is configured to store a plurality of auscultation positions on the living body surface, and an auscultation sequence, wherein
the displaying section is configured to display results of analysis corresponding to the plurality of auscultation positions based on the auscultation sequence.

19. The electronic auscultation system according to any one of claims 16 to 18, further comprising:
a sensor which is adapted to be attached to the subject; and
a biological information acquiring section which is configured to acquire biological information of the subject through the sensor, wherein
the displaying section is configured to display the biological information and the analysis result in a temporally synchronized manner.
